Europäisches Patentamt

European Patent Office

Office européen des brevets

(19)

(11) **EP 0 813 865 B1**

(12) **EUROPÄISCHE PATENTSCHRIFT**

(45) Veröffentlichungstag und Bekanntmachung des Hinweises auf die Patenterteilung:
**19.09.2001   Patentblatt 2001/38**

(51) Int Cl.⁷: **A61K 9/70**

(21) Anmeldenummer: **97113342.6**

(22) Anmeldetag: **20.04.1994**

(54) **Wirkstoffpflaster**

Drug patch

Pansement avec une substance active

(84) Benannte Vertragsstaaten:
**AT BE CH DE DK ES FR GB GR IE IT LI LU MC NL PT SE**

(30) Priorität: **20.04.1993  DE 4312818**
**18.11.1993  DE 4339400**

(43) Veröffentlichungstag der Anmeldung:
**29.12.1997   Patentblatt 1997/52**

(62) Dokumentnummer(n) der früheren Anmeldung(en) nach Art. 76 EPÜ:
**94913616.2 / 0 695 177**

(73) Patentinhaber: **HEXAL AG**
**D-83607 Holzkirchen (DE)**

(72) Erfinder:
• **Fischer, Wilfried, Dr.**
**83607 Holzkirchen (DE)**

• **Klokkers, Karin, Dr.**
**83607 Holzkirchen (DE)**

(74) Vertreter: **Boeters, Hans Dietrich, Dr. et al**
**Patentanwälte Boeters & Bauer,**
**Bereiteranger 15**
**81541 München (DE)**

(56) Entgegenhaltungen:
**WO-A-89/09051          WO-A-92/20339**

• **DATABASE WPI Week 9025 Derwent Publications Ltd., London, GB; AN 90-189690 XP002042866 & JP 02 124 821 A (TOYAMA CHEM KK) , 14.Mai 1990**
• **DATABASE WPI Week 8448 Derwent Publications Ltd., London, GB; AN 84-297371 XP002042867 & JP 59 184 121 A (NITTO ELECTRIC IND KK) , 19.Oktober 1984**

## Beschreibung

Die transdermale Anwendung, das heißt systemische Zufuhr nach dermaler Applikation, von Arzneistoffen, wie Estradiol, Nitroglycerin, Nicotin, Selegilin, Piroxicam, Estradiolvalerat, Norethistosteron, Norethistosteronacetat, Levonorgesterel etc., um nur einige exemplarisch zu nennen, hat gegenüber der oralen Gabe den Vorteil der Resorption unter Umgehung des Magen-Darm-Traktes und damit der Vermeidung der Metabolisierung der Arzneistoffe bei der ersten Leberpassage. Durch die hepatische Metabolisierung kann es zu unerwünschten Konzentrationsverhältnissen zwischen Muttersubstanz und Metaboliten kommen. Ein weiterer Vorteil der transdermalen Gabe liegt in der Gleichmäßigkeit der Blutspiegel nach Resorption der Wirkstoffe durch die Haut, da Einflüsse, wie unterschiedlicher Füllungsgrad des Magen-Darm-Traktes, Wechselwirkungen mit Nahrungsmitteln oder Einschränkung bzw. Beschleunigung der Darmmobilität, wie sie bei peroraler Gabe auftreten, nicht vorliegen. Im Falle eventuell auftretender wirkstoffbedingter Nebenwirkungen kann ein technisches System zur systemischen Wirkstoffzufuhr über die Haut (transdermales therapeutisches System TTS, transdermal (drug) delivery system, TD(D)S, im folgenden Wirkstoffpflaster oder Pflaster genannt, von der Haut entfernt und damit die Wirkstoffzufuhr sofort unterbrochen werden. Die derzeit im Handel befindlichen Systeme, beispielsweise für Estradiol, die Kombination aus Estradiol und Norethisteronacetat, Nitroglycerin und Scopolamin, oder Systeme, die sich in klinischer Erprobung befinden, wie Systeme für Testosteron, enthalten überwiegend die Wirkstoffe gelöst in alkoholischer Lösung. Alkohol in direktem längeren Kontakt mit der Haut führt in vielen Fällen zu Hautirritationen oder sogar zu Allergien, die zu einem Abbruch der Therapie zwingen. Dies war der Grund für die weiter unten beschriebenen Systeme mit alternativen Resorptionsförderern oder übersättigten Systemen (im Lagerzustand) ohne Absorptionsförderer. Gerade die übersättigten Systeme sind jedoch persekalisch instabil. In diesen Pflastern kann der oder die Wirkstoffe unvorhersehbar rekristallisieren und damit die Permeation dieser Wirkstoffe durch die Haut in unvorhersehbarer Weise reduziert werden. Damit verbunden ist dann eine nicht ausreichende Permeation des Wirkstoffes durch die Haut, die damit unter Umständen zu einer Unterschreitung der therapeutisch notwendigen Blutspiegelkonzentration führen kann. Übersättigte Pflaster sind metastabil und damit prinzipiell instabil.

In der Regel verändern chemische Resorptionsförderer, als bekanntestes Beispiel möge Azone dienen, die Struktur der Haut derart, daß die Haut ihre natürliche Barrierefunktion verliert. Die Resorptionsförderer gehen Wechselwirkungen mit Hautkomponenten ein, so daß in vielen Fällen Hautirritationen und allergische Erscheinungen resultieren. Es besteht also ein dringender Bedarf an hautfreundlichen, therapeutisch sicheren und stabilen Wirkstoffpflastern für Arzneistoffe.

## Stand der Technik

Entsprechend dem Stand der Technik können die Pflaster zur systemischen Hormonzufuhr in drei Klassen eingeteilt werden:

1. Systeme enthaltend Absorptionsförderer

2. Systeme ohne Absorptionsförderer

3. Systeme enthaltend hydrophile quellbare Polymere zur Stabilisierung der Übersättigung

## 1. Pflaster mit Absorptionsförderern

Pflaster mit Absorptionsförderern stellen mit Abstand die größte Gruppe dar, da in der technischen Literatur z. B. Estradiol oder gestagene Hormone als schwer permeierende Substanzen beschrieben wurden.

EP-A-0 474 647 beschreibt die Verwendung von hydratisiertem Aluminiumsilikat zur Erhöhung der Klebkraft. Als Lösemittel wird ein Glycol verwendet, das auch als Absorptionsförderer dient. Glycole haben neben ihrer resorptionsfördernden Eigenschaft die Nebenwirkung der Hautaustrocknung. US-A-5 023 084 beansprucht Progesterone und Oestrogene in getrennten Schichten, jeweils mit dem Absorptionsförderer n-Decanol. Decanol ist ein pharmazeutisch nicht zugelassener hautreizender und unangenehm riechender Stoff. EP-A-0 371 496 (= US SN 278 625) beschreibt die Verwendung von Ölsäure, einem linearen Alkohol-Milchsäure-Ester, Dipropylenglycol oder N-Methyl-2-pyrrolidon als Absorptionsförderer. US-A-5 006 342 beansprucht die Verwendung von Fentanyl neben Estradiol mit hydrophoben Polymeren in Kombination mit Absorptionsförderern, wie Propylenglycolmonolaurat. US-A-4 906 475 beschreibt die Verwendung eines Lösemittels, wie PEG, in Kombination mit einem Absorptionsbeschleuniger, wie Polysorbat 80. Polysorbat 80 ist ein Tensid, das den Aufbau der Haut zerstören und damit Hautschädigungen hervorrufen kann. EP-A-0 402 407 (= US SN 160 635) beschreibt die Verwendung eines kombinierten Absorptionsförderers aus Isopropylmyristat und Laurinmonoglycerid. EP-A-0 430 491 (= AR SN 315 479) beansprucht die Verwendung von Absorptionsförderern, wie ungesättigte Fettsäuren und deren Ester sowie Glycerin und Alkylen-1,2-diole. US-A-5 053 227 und 5 059 426 beanspruchen die Verwendung von Diethylenglycol-monomethylether allein oder in Kombination mit einem Alkylester. US-A-4 956 171 beansprucht Verwendung eines kombinierten Enhancers, bestehend aus Sucrosecocoat und Methyllaurat. US-A-4 996 199 beansprucht die Verwendung tertiärer Amine und Amide als Absorptionsförderer. US-A-4 906 169 beinhaltet ein mehrschichtiges Pflaster mit einer estradiolhaltigen Schicht und einer gestagenhaltigen Schicht, die zusätzlich einen Absorptionsförderer enthält.

Absorptionsförderer der oben beschriebenen Substanzklassen sind potentiell insofern hautschädigend, als sie zur Penetration von Arzneistoffen in die Haut deren Struktur stören müssen, um sie durchlässiger zu machen. Das hat in vielen Fällen Hautreizungen bis hin zu Sensibilisierungen zur Folge. Selbst Ethylalkohol kann bei intensivem Kontakt Allergien auslösen. Keines der beschriebenen Pflaster enthält Substanzen zur Vermeidung von Hautirritationen.

**2. Pflaster ohne Absorptionsförderer**

Das japanische Patent JP-A-02.229 114 beschreibt ein Pflaster, bei dem Estradiol in Polyvinylpyrrolidon (PVP) in der Massenkonzentration 3 bis 17 % enthalten ist. Die PVP-Schicht ist dabei in direktem Kontakt mit der Haut in einer Fläche von 2 bis 40 cm$^2$. Das PVP ist kein druckempfindlicher Haftkleber. Dieses Pflaster benötigt zur Fixierung auf der Haut zusätzlich einen Klebstoffrand. EP-A-0 346 211 (= JP-A-02.196 714, 02.233 617, 03.017 018, 03.044 326 und 03.044 327) beanspruchen die Verwendung eines Copolymers aus 2-Ethylhexylacrylat und N-Vinyl-2-pyrrolidon ohne Absorptionsförderer. EP-A-0 272 918 (= US SN 945 389) beschreibt die Verwendung eines makroporösen Schaumes, in dem Wirkstoff immobilisiert vorliegt. EP-A-0 409 383 (= US SN 461 676) beschreibt ein östrogenhaltiges Pflaster im Konzentrationsbereich von 0,01 bis 1 % eines Östrogens in Kombination mit einem wasserunlöslichen Vinylpyrrolidon zur retardierten Abgabe des Wirkstoffes an die Haut. US-A-4 994 267 beschreibt eine Mischung eines synthetischen oder natürlichen Gummis in Kombination mit Ethylen-Vinylacetat-Copolymer und Acrylat. AU-A-91.76 582 (= JP SN 90.202 409) beschreibt die Verwendung eines Acrylatklebers in Kombination mit einer Polyesterträgerfolie mit einer Schichtdicke von 0.5 bis 4.9 µm und einer Dehnungsfähigkeit vom Faktor 1 bis 5 in einer Flächenrichtung senkrecht zur anderen. Die Erfinder beanspruchen eine ausreichende Wirkstoffabsorption lediglich durch den mechanischen Effekt, daß das Pflaster sich durch die dehnbare Folie optimal an die Haut anschmiegt. EP-A-0 416 842 (= US SN 405 630) beschreibt ausdrücklich die Verwendung von Acrylatcopolymeren ohne Absorptionsförderer, in denen Wirkstoffe, vorzugsweise Östrogene oder Norethisteron oder Norethisteronacetat, allein oder in Kombination enthalten sind. Diese oben beschriebenen Pflaster stellen lediglich Arzneistoffträger dar, die keinerlei Resorptionssteuerung zulassen. Die Haut kann nicht gezielt für den schwer permeierenden Wirkstoff Norethisteronacetat und/oder Estradiol permeabel gemacht werden, so daß die sich einstellenden Blutspiegel sehr stark vom jeweiligen Hauttyp und -zustand abhängig sind. Solche Systeme erzeugen sehr stark schwankende Blutspiegel und können nicht die erforderliche therapeutische Zuverlässigkeit garantieren.

Die gerade beschriebenen prinzipiellen Unzulänglichkeiten weisen auch übersättigte Pflaster auf, die im folgenden beschrieben sind.

**3. Übersättigte Systeme**

EP-A-0 186 019 beansprucht die Verwendung eines wasserquellbaren Polymers in z. B. Polyisobutylenmatrix zur Kristallisationsverzögerung des oberhalb seiner Sättigungskonzentration vorliegenden Wirkstoffes. Hierdurch soll die thermodynamische Aktivität des Estradiol gesteigert und die Hautpermeation gefördert werden, um auf Resorptionsförderer verzichten zu können. Die starke Streuung der Blutspiegel kann aber auch hierdurch nicht vermieden werden. Auch hierbei werden keine Maßnahmen zur Reduktion von Hautirritationen vorgenommen, die bei druckempfindlichen Haftklebern erfahrungsgemäß immer auftreten können.

**Beschreibung der Erfindung**

Der vorliegenden Erfindung lag nun die Aufgabe zugrunde, ein zuverlässig wirkendes, dabei hautfreundliches und wirtschaftlich herzustellendes Pflaster für die systemische Applikation von Wirkstoffen zu entwickeln. Beispiele für Wirkstoffe sind im folgenden in 16 Gruppen zusammengefaßt.

Das Pflaster sollte aus medizinisch gebräuchlichen Klebstoffen und sonstigen pharmokopoeüblichen Hilfsstoffen (ohne hautschädigende oder potentiell hautschädigende Eigenschaften) bestehen. Das Pflaster sollte dabei möglichst hoch mit Wirkstoffen beladbar sein, ohne seine Klebkraft einzubüßen, um über möglichst lange Zeit gleichmäßig hohe Blutspiegel zu erzeugen. Hierzu wurden beispielhaft Wirkstoffe, wie 17-β-Estradiol, Norethisteronacetat u. a., in den medizinisch verwendbaren Acrylatklebern oder Silikonklebern gelöst und die Klebkraft auf menschlicher Haut, die in vitro Wirkstofffreisetzung nach dem amerikanischen Arzneibuch USP XXII, die in vitro Hautpermeation durch die isolierte Haut von Nacktmäusen, bzw. durch Klebstoffmembranen, die Rekristallisation der inkorporierten Wirkstoffe sowie der Wassergehalt von Pflastern mittels eines empfindlichen elektrometrischen Verfahrens untersucht.

Dabei wurde nun gefunden, daß medizinisch gebräuchliche druckempfindliche Acrylathaftkleber, wie z. B. ein Acrylat-Copolymer aus (Duro-Tak 1753, Fa. National Starch & Chemical, D-Neustadt), zwar gute Löseeigenschaften für Estradiol und Norethisteronacetat haben, die Anfangsklebkraft und die Langzeitklebkraft aber zu wünschen übrig lassen. Druckempfindliche Silikonhaftkleber sind weicher und haben eine hohe Anfangsklebkraft. Das Lösevermögen für die o. g. Hormone ist jedoch sehr viel schlechter, so kristallisiert z. B. das Estradiol einer 2-proz. Lösung in einem Silikonkleber (Bio-PSA X7-4602, Fa. Dow Corning GmbH, D-Meerbusch) innerhalb von ca. 2 Wochen bei Raumtemperatur wieder aus. Da nur gelöster Wirkstoff für die Hautpermeation verfügbar ist, müssen die Pflaster gegenüber Rekristallisationen stabil sein.

Um nun aber eine hohe Diffusionsfähigkeit der inkorporierten Wirkstoffe zu erreichen, sollte das auf der Haut klebende Pflaster den Wirkstoffen eine hohe thermodynamische Aktivität verleihen. Dieses wird z. B., wie bereits dargelegt, durch eine über die Löslichkeit der Wirkstoffe in den Polymeren hinausgehende Konzentration erreicht. Diese Systeme sind übersättigt und damit physikalisch instabil. Die Rekristallisation der Wirkstoffe kann nicht kontrolliert werden und damit wird die thermodynamische Aktivität unkontrolliert während der Lagerung solcher Pflaster abnehmen.

Völlig überraschend ist nun die Lehre, daß Mischungen aus druckempfindlichen Acrylat- und/oder Silikonklebern mit natürlichem $\alpha$-Tocopherol eine ausgezeichnete Anfangs- und Dauerhaftung auf Haut aufweisen und gleichzeitig nach Aufbringen auf die Haut den eingearbeiteten Wirkstoffen eine hohe thermodynamische Aktivität verleihen. $\alpha$-Tocopherol ist als Vitamin E mit seinen pharmakologischen Wirkungen bekannt, jedoch nicht als Harz zur Erhöhung der Klebefähigkeit von Polymeren. Dies ist umso erstaunlicher, als Zumischung von Ölen, wie Silikonöl, in gleichen Mengen zu einem nahezu völligen Verlust der Klebkraft führt.

Dabei sind im Falle der Mischung der Polymere beide Polymere miteinander in der getrockneten Klebematrix inkompatibel, sie bilden ein emulsionsartiges trübes Polymergemisch, während die Einzelkomponenten klare Klebematrices bilden. Die Beladung der Matrix mit Wirkstoff(en) ist dabei vom Mischungsverhältnis der Polymeren und deren Mischung mit $\alpha$-Tocopherol abhängig. Mit zunehmendem Silikonanteil nimmt die Löslichkeit in der getrockneten Matrix ab, die Gesamtmenge der Wirkstoffe, die sich in der Klebstoffmatrix lösen, läßt sich durch $\alpha$-Tocopherol jedoch wieder erhöhen. Mit der Veränderung der Zusammensetzung der beiden Klebstofftypen untereinander oder der Einzelklebstoffe mit $\alpha$-Tocopherol kann also eine definierte Maximallöslichkeit eingestellt werden. Dies hat wiederum zur Folge, daß die Dauer, in der Wirkstoff an die Haut abgegeben werden kann, mit diesem Prinzip gesteuert werden kann. Falls auf die Anwendung des Polymergemisches verzichtet wird und nur das Polyacrylat eingesetzt wird, so kann die Klebkraft des schlecht haftenden Polymers durch $\alpha$-Tocopherol allein gesteigert werden. Dies kann, abhängig von Polymer und Wirkstoff, ausreichen, um die Klebkraft so zu steigern und auf die Zumischung von Silikon zu verzichten.

Die bisher beschriebenen erfindungsgemäßen Pflasterzusammensetzungen sind höchstens wirkstoffgesättigte Systeme, die lagerstabil sind und nicht zu Kristallisationen führen. Weiterhin überraschend war nun die Erkenntnis, daß die so beschriebenen Pflaster erst auf der Haut übersättigte Zustände ergeben und somit die thermodynamische Aktivität der Wirkstoffe über die der Lagerform hinaus ansteigt. Dabei ist der Grad der Übersättigung vom Ausgangswassergehalt der Pflaster und dem $\alpha$-Tocopherolgehalt abhängig. Darüber hinaus kann die Übersättigung durch Trägerfolien unterschiedlicher "Dichtigkeit" beeinflußt werden und damit die thermodynamische Aktivität der Wirkstoffe.

Völlig unerwartet war dabei die Tatsache, daß der Wirkstoffgehalt in der Polymermatrix sehr stark vom Restwassergehalt des Polymers im Promillbereich abhängt. Für die weiteren Ausführungen möge Estradiol als Beispiel für geeignete Arzneistoffe stehen. So kann zum Beispiel eine Matrix bestehend aus Acrylatpolymer (Durotak 1753), die 7,5 % $\alpha$-Tocopherol enthält, bei einem Wassergehalt von 1,2 % etwa 2,5 % Estradiol lösen, während bei einem Wassergehalt von etwa 3,9 % nur noch 1,3 % Estradiol in der so hydratisierten Matrix löslich sind. Die Wasserbestimmung der Matrix wurde dabei mit einer modifizierten elektrometrischen Karl-Fischer-Bestimmungsmethode durchgeführt.

Die Löslichkeit des Estradiol in der Matrix wurde auf zwei Arten bestimmt:

1. Löslichkeitsbestimmung in der "trockenen" Matrix

Da die Löslichkeitsbestimmung von Wirkstoffen in hochviskosen Lösungsmitteln, wie den hier verwendeten Klebstoffen, nicht wie bei flüssigen Systemen über eine einfache Konzentrationsmessung der mit Bodensatz im Gleichgewicht stehenden Phase des Lösungsmittels möglich ist, wurden Klebstoffmatrices mit unterschiedlichen Wirkstoffgehalten durch Beschichten eines silikonisierten Trägerfilms mit der Wirkstoff-Klebstoff-Lösung und anschließendem Abdampfen des Lösungsmittels hergestellt. In die so erhaltenen Klebstoffmatrices wurden Estradiolimpfkristalle mit genau definierter Länge eingebracht und die Änderung der Kristalldimensionen über die Zeit ausgemessen. Kommt es im Beobachtungszeitraum zu keinerlei Größenänderungen des Kristalles befindet sich die Wirkstoffkonzentration in der Matrix in der Nähe der Sättigungskonzentration. Die so ermittelte Löslichkeit für Estradiol beträgt in einem "trokkenen" System, das heißt Wassergehalt < 5 %, ca. 2,5 Gew.-%.

2. Bestimmung der Estradiolkonzentration in einer vollständig hydratisierten Matrix

Der Flux J einer Substanz durch eine Membran definierter Schichtdicke ergibt sich aus der vereinfachten Gleichung 1 zu

$$J = \frac{dM}{dt} = \frac{D \times A \times K \times C_0}{h} \tag{1}$$

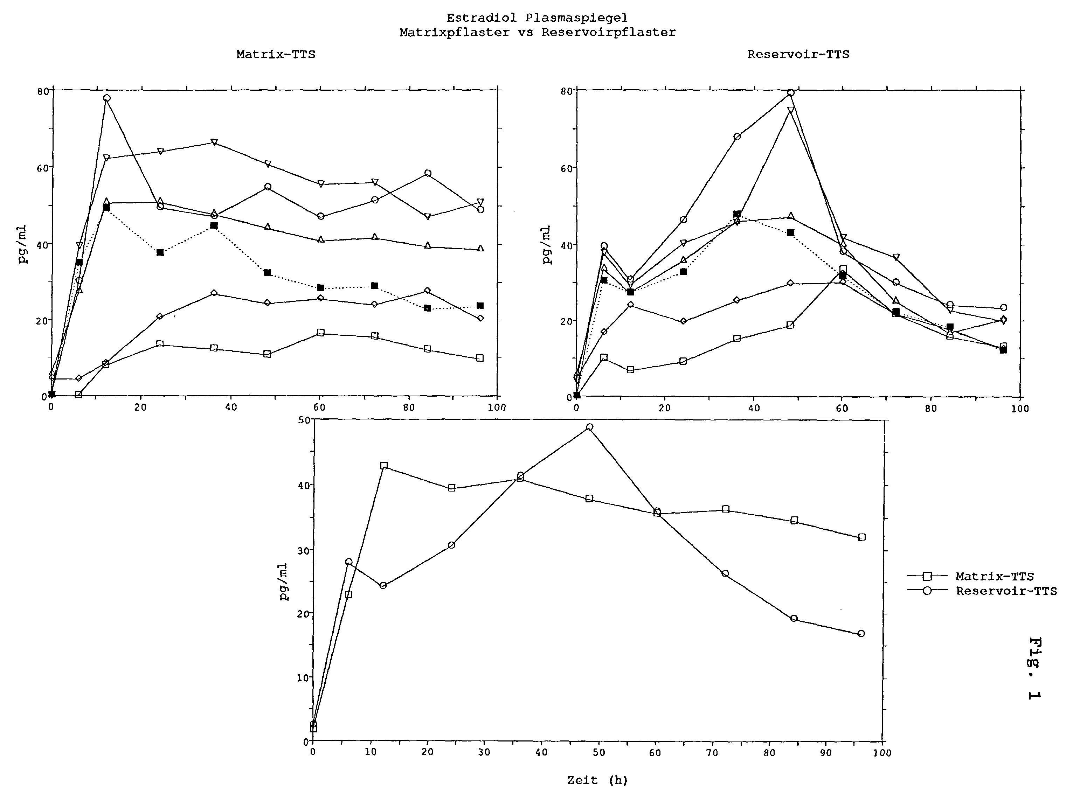

Diese Beziehung gibt den konstanten Flux im Gleichgewichtszustand eines Diffusionsexperiments an. Demnach ist die pro Zeit (t) durch die Membran diffundierende Menge (M) direkt proportional zur Konzentration auf der Donorseite der Membran (Co). Die weiteren Parameter sind D Diffusionskoeffizient, A Diffusionsfläche, K Verteilungskoeffizient zwischen Donor und Membran, h Dicke der Membran. Die Variable J ist aus dem Diffusionsdiagramm der **Abb. 1** aus der Steigung des geraden Kurvenabschnittes bestimmbar. A - die Diffusionsfläche - und h - die Membrandicke - sind direkt messbar, so daß zur Bestimmung der Konzentration noch der Diffusionskoeffizient und der Verteilungskoeffizient bestimmt werden müssen.

Der Diffusionskoeffizient D kann entsprechend der Gleichung

$$t_{lag} = \frac{h^2}{6 \times D} \tag{2}$$

aus der Lagtime und der Schichtdicke der Membran bestimmt werden, so daß letztendlich noch der Verteilungskoeffizient K bestimmt werden muß. Für den Fall, daß das Donorkompartiment und die Membran aus dem gleichen Material bestehen, kann näherungsweise der Verteilungskoeffizient K = 1 angenommen werden. In der Praxis kann man so vorgehen, daß eine gesättigte Klebstoffschicht, die einen Überschuß des Wirkstoffes in ungelöster Form enthält, auf eine wirkstofffreie Membran aus dem gleichen Material bekannter Schichtdicke geklebt wird. Danach wird in einer Diffusionszelle der Übergang des Wirkstoffes durch die wirkstofffreie Membran gegen die Zeit gemessen. Aus der dabei entstehenden typischen Diffusionskurve können die oben beschriebenen Parameter leicht berechnet werden. Da die Membran sich durch den Kontakt mit dem Akzeptormedium Wasser vollständig hydratisiert hat, ist die erhaltene Sättigungskonzentration die einer maximal wasserhaltigen Klebstoffmatrix. In der hydratisierten, zu Beginn des Experimentes wirkstofffreien Membran kann sich im Kontakt mit der gesättigten Klebstoffschicht höchstens etwa 1,3 % Estradiol lösen. Dies gilt für eine Zusammensetzung der Matrix von 92,5 % Durotak 1753 und 7,5 % Copherol. Brächte man nun eine Wirkstoffmatrix, die im trockenen Zustand 2,5 % Estradiol aufnimmt, in den maximal hydratisierten Zustand, so würde durch die Reduktion der Löslichkeit sich dadurch, daß die Kristallisation durch hohe Viskosität des Klebers kinetisch gehemmt ist, eine Übersättigung von ca. 95 % ergeben. Die hohe Viskosität der Klebstoffmatrix kann diesen übersättigten Zustand über mehrere Tage stabil erhalten. Wird aus einer derart beschriebenen Matrix ein Pflaster hergestellt, so kann die Matrix nach dem Aufkleben auf die Haut durch die Perspiratio Insensibilis hydratisiert und damit hinsichtlich des Wirkstoffes stark übersättigt werden. Eine Übersättigung des TTS bedingt - wie eingangs erläutert - eine Erhöhung der thermodynamischen Aktivität und damit eine Erhöhung der die Diffusion antreibenden Kraft. Die Übersättigung des hydratisierten Systems nach Applikation ist also erwünscht und auch notwendig, um schwer permeierenden Arzneistoffen eine hohe thermodynamische Aktivität zu verleihen. Dadurch gelingt es, unter Verzicht auf Resorptionsförderer, auch schwer permeierende Stoffe durch die Haut diffundieren zu lassen. Im Beispielteil wird gezeigt, wie anhand von in vitro Hautpermeationsstudien belegt wird, daß die Permeation im Verhältnis zu einem System mit einem Absorptionsförderer mindestens gleich hohe Permeationsraten erreicht. Damit hat das erfindungsgemäß hergestellte System den Vorteil, daß es während des Lagerzustandes gesättigt oder in der Nähe der Sättigungskonzentration liegt und damit stabil ist und erst nach dem Aufkleben auf die Haut durch Wasseraufnahme die erhöhte thermodynamische Aktivität erhält. Die Wasseraufnahme der Matrix kann dabei durch Variation des $\alpha$-Tocopherolgehaltes beeinflußt werden. So enthält eine Matrix, bestehend aus 2,5 % Estradiol, 5 % $\alpha$-Tocopherol und 92,5 % Acrylatkleber, direkt nach der Herstellung etwa 2,6 % Wasser. Eine Matrix, bestehend aus 2,5 % Estradiol, 7,5 % $\alpha$-Tocopherol und 90 % Acrylatkleber, enthält dagegen nach Herstellung und Trocknung unter identischen Bedingungen nur 1,2 % Wasser. Die maximale Löslichkeit von Estradiol in einer Matrix aus 92,75 % Acrylatkleber und 5 % $\alpha$-Tocopherol ist etwa 2,25 % bei einer Restfeuchte von ca. 2,6 % Wasser. Eine Matrix aus 90 % Acryatkleber und 7,5 % $\alpha$-Tocopherol löst etwa 2,5 % Estradiol bei einem Wassergehalt von ca. 1,2 %. Die Löslichkeit in hydratisierten Matrices beträgt dagegen etwa 1,3 %. Bezogen auf die handelsübliche Dosierung von 4 mg Estradiol pro Pflaster ergibt sich im ersten Fall eine Übersättigung von 1,69 mg, im zweiten Fall von 1,92 mg. Dies entspricht einer Erhöhung der Übersättigung und damit der thermodynamischen Aktivität um etwa 10 %. $\alpha$-Tocopherol kann somit den Grad der Übersättigung hydratisierter Matrices und damit die Diffusion von Wirkstoffen durch die Haut bestimmen.

Eine weitere Möglichkeit zur Beeinflussung der Wasseraufnahme der Matrix und damit zur Steuerung des Grades der Übersättigung bietet die Verwendung von Trägerfolien, die nach Aufkleben der Pflaster auf die Haut die Wasserverdunstung in unterschiedlichem Grad beeinflussen. Die maximale Hydratation, die im obigen Fall beschrieben wurde, erhält man durch Verwendung von okklusiven Folien. Hierzu gehören beispielsweise Polyester oder Polypropylen-Polyethylenfolien. Mißt man die Wasserabgabe der Systeme nach Aufleben auf die Haut an die Umgebung, so stellt sich beispielsweise nach Applikation auf den Unterarm bei einem transepidermalen Wasserverlust (TEWL) von 7,1 g/m$^2$/h unter Verwendung von Polyterephthalsäureester-Folie (PE, 19 µm) ein TEWL von 3,1 g/m$^2$/h ein. Nach fünfstündiger Applikation eines solchen Pflasters steigt unmittelbar nach dem Abziehen des Pflasters von der Haut der TEWL auf circa 40 bis 50 g/m$^2$/h an. Die Haut ist also stark hydratisiert. Ein Pflaster, das Polyurethan (PU, 40 µm) als Abdeckfolie enthält, reduziert dagegen den TEWL auf circa 5,9 g/m$^2$/h. Nach Abziehen steigt der Wert nur auf circa 11 g/m$^2$/h. Die Haut 5 unter einem solchen Pflaster ist gegenüber dem Normalzustand dementsprechend geringer hydratisiert. Eine durch wasserdampfdurchlässige Folie abgedeckte Matrix wird dementsprechend nicht so viel Wasser aufnehmen wie eine vollständig abgedeckte Matrix. Damit wird der Grad der Übersättigung sich vom Maximalwert entfernen und die Permeation sinken.

In der Praxis kann in der bevorzugten Ausführung der Erfindung das Acrylatpolymer ein beliebiges Homopolymer, Copolymer, Terpolymer, bestehend aus verschiedenen Acrylsäurederivaten, sein. In solch einer bevorzugten Ausführung macht das Acrylsäurepolymer von ca. 2 bis ca. 95 % des gesamten Gewichts in der gesamten dermalen Zusammensetzung und vorzugsweise ca. 2 bis ca. 90 % aus. Die Menge des Acrylatpolymers ist abhängig von der Menge und dem Typ des verwendeten Arzneistoffes, der in das verwendete Arzneimittel eingearbeitet wird.

Die Acrylatpolymere dieser Erfindung sind Polymere eines oder mehrerer Monomere von Acrylsäuren und anderen copolymerisierbaren Monomeren. Außerdem beinhalten die Acrylatpolymere Copolymere von Alkylacrylaten und/oder Methacrylaten und/oder copolymerisierbaren sekundären Monomeren oder Monomeren mit funktionellen Gruppen. Verändert man den Betrag jeder Sorte, die als Monomer hinzugefügt ist, können die kohäsiven Eigenschaften und Lösungseigenschaften der daraus resultierenden Acrylatpolymere verändert werden. Im allgemeinen besteht das Acrylatpolymer aus mindestens 50 Gew.-% eines Acrylate- oder Alkylacrylat-Monomers, 0 bis 20 % eines funktionellen Monomers, copolymerisierbar mit Acrylat, und 0 bis 40 % eines anderen Monomeren.

Im folgenden sind Acrylatmonomere aufgeführt, die mit Acrylsäure, Methacrylsäure, Butylmetharylat, Hexylacrylat, Hexylmethacrylat, Iscooctylacrylat, Isooctylmethacrylat, 2-Ethylhexylacrylat, 2-Ethylhexylmethacrylat, Decylacrylat, Decylmethacrylat, Dodecylacrylat, Dodecylmethacrylat, Tridecylacrylat und Tridecylmethacrylat verwendet werden können.

Folgende funktionelle Monomere, copolymerisierbar mit den oben genannten Alkylacrylaten oder -methacrylaten, können einschließlich mit Acrylsäure, Methacrylsäure, Maleinsäure, Maleinanhydrid, Hydroxyethylacrylat, Hydroxypropylacrylat, Acrylamid, Dimethylacrylamid, Acrylnitril, Dimethylaminoethylacrylat, Dimethylaminoethylmethacrylat, tert-Butylaminoethylacrylat, tert-Butylaminoethylmethacrylat, Methoxyethylacrylat und Methoxyethylmethacrylat eingesetzt werden.

Weitere Einzelheiten und Beispiele für klebende Acrylate, welche für die Erfindung geeignet sind, sind in Satas Handbook of Pressure Sensitive Adhesive Technology "Acrylic Adhesives", 2nd ed., pp. 396 - 456 (D. Satas, ed.), Van Nostrand Reinhold, New York (1989) beschrieben.

Passende klebende Acrylate sind im Handel unter dem Warenzeichen DuroTak erhältlich und beinhalten den Polyacrylatkleber.

Passende Polysiloxane beinhalten druckempfindliche Silicon-Kleber, welche auf zwei Hauptbestandteilen basieren: Ein Polymer oder Klebstoff und ein tackerhöhendes Harz. Der Polysiloxankleber ist gewöhnlich mit einem Vernetzer für den Kleber, typischerweise einem hochmolekularen Polydiorganosiloxan, und mit dem Harz zubereitet, um über ein angemessenes organisches Lösungsmittel eine dreidimensionale Silicatstruktur zu ergeben. Die Zumischung des Harzes zu Polymer ist der wichtigste Faktor, um die physikalischen Eigenschaften der polysiloxanen Kleber zu ändern. Sobieski, et al., "Silicone Pressure Sensitive Adhesives", Handbook of Pressure Sensitive Adhesive Technology, 2nd ed., pp. 508 - 517 (D. Satas, ed.), Van Nostrand Reinhold, New York (1989).

Passende druckempfindliche Silikonkleber sind im Handel unter dem Warenzeichen BIO-PSA X7 erhältlich.

Beispielhafte aktive Arzneimittel die bei dem Pflaster dieser Erfindung enthalten sein können sind:

1. Kreislaufwirksame Arzneistoffe, wie beispielsweise organische Nitrate, wie Nitroglycerinisosorbiddinitrat; Procainamid; Thiazide; Dihydropyridine, wie Nifedipin oder Nicardipin; Betablocker, wie Timolol oder Propranolol; ACE-Hemmer, wie Enalapril, Captopril oder Lisinopril; oder Alpha-2-Blocker, wie Clonidin oder Prazosin.

2. Androgene Steroide, wie Testosteron, Methyltestosteron oder Fluoximesteron.

3. Oestrogene, wie Oestradiolester, Estradiolpropionat, $17$-$\beta$-Estradiol, $17$-$\beta$-Estradiolvalerat, Estron, Mestranol, Estriol, $17$-$\beta$-Ethinylestradiol oder Diethylstilbestrol.

4. Progestagene Hormone, wie Progesteron, 19-Nor-Progesteron, Norethisteron, Norethisteronacetat, Melengestrol, Chlormadinon, Ethisteron, Medroxiprogesteronacetat, Hydroxiprogesteroncaproat, Ethynodioldiacetat, $17$-$\alpha$-Hydroxiprogesteron, Norgestrel und andere.

5. Wirkstoffe mit Wirkung auf das zentrale Nervensystem, wie beispielsweise Sedativa, Hypnotika, Anxiolytika, Antidepressiva, Analgetika und Anaesthetika, wie Buprenorphin, Naloxon, Haloperidol, Flufenacin, Barbitale, Lidocain, Mepivacain, Fentanyl, Suventanil oder Nikotin.

6. Mittel zur Behandlung von Parkinsonismus, wie Selegelinsalze oder Selegilinbase, Bromocriptin, Lisurid und andere.

7. Entzündungshemmende Wirkstoffe, wie Hydrocortison, Cortison, Dexamethason, Triamcinolon, Prednisolon, Ibuprofen, Naproxen, Fenoprofen, Flurbiprofen, Indoprofen, Ketoprofen, Piroxicam, Diflunisal und andere.

8. Antihistaminika, wie Dimenhydrinat, Perfenacin, Prometacin, Terfenadin und andere.

9. Wirkstoffe mit Wirkung auf den respiratorischen Trakt, wie Theophyllin oder $\beta_2$-adrenergische Agonisten, wie Albuterol, Terbutalin, Fenoterol, Salbutamol und andere.

10. Sympatomimetika, wie Dopamin, Phenylpropanolamin, Phenylefrin und andere.

11. Antimuskarinika, wie Atropin, Scopolamin, Homatropin, Benzatropin und andere.

12. Dermatologische Wirkstoffe, wie Vitamin A, Cyclosporin, Dexpanthenol und andere.

13. Prostaglandine, wie Prostaglandin E1, Prostaglandin E2, Prostaglandin F2 und Analoge.

14. Antiöstrogene, wie Tamoxifen, 3-Hydroxi- und 4-Hydroxitamoxifen.

15. Antimigränemittel, wie Dihydroergotamin und Pizotylin.

16. Antiulzarativa, wie Misoprostol, Omeprazol, Enprostil oder Ranitidin.

Die im Pflaster enthaltene absolute Wirkstoffmenge bestimmt die Zeitspanne, in der eine kontinuierliche Zufuhr in den Organismus aufrecht erhalten wird. Deshalb ist eine möglichst hohe Beladung des Polymersystems mit Wirkstoffen dann wünschenswert, wenn die Applikationszeit eines Pflasters lang ist, d.h. mehrere Tage bis zu einer Woche beträgt.

Um die Löslichkeit von Wirkstoffen weiter zu erhöhen, wurden diverse Zuschlagstoffe am Beispiel eines Pflasters, enthaltend zwei Wirkstoffe, Estradiol und Norethisteronacetat, im Vergleich zu α-Tocopherol erprobt, wie Tenside, Öle oder schwer verdampfende Lösemittel. Tenside waren z. B. Natriumlaurylsulfat, Polyethylen(20)sorbitanmonooleat, Saccharosemonomyristat (Sucroseester) oder andere toxikologisch unbedenkliche Substanzen. Alle Zusätze führten zu einer beschleunigten Rekristallisation der inkorporierten Wirkstoffe. Pharmazeutisch gebräuchliche Öle, wie Ölsäureoleylester, Olivenöl, Sojaöl u.a., sowie Lösemittel, wie Propylenglycol, Glycerin, Polyethylenglycol oder Diethylenglycol, konnten zwar die Rekristallisationszeit der Wirkstoffe hinauszögern, nicht jedoch die Kristallisation unterbinden (Tab. 1). Im Vergleich dazu zeigt die Tab. 2 die Wirkung von α-Tocopherol auf die Löslichkeit. So können 10 % α-Tocopherol bis zu 2,5 % Estradiol und 10 % Norethisteronacetat in der Matrix stabilisieren. Diese Tatsache ist äußerst ungewöhnlich, da selbst gute, bekannte Lösemittel, wie Ölsäureoleylester oder Diethylenglycol eine mit Abstand schlechtere Wirkung hatten. Die Klebematrix bestand in allen Fällen aus 80 % des Acrylat- und 20 % des Silikonklebers. Der Gehalt an Wirkstoffen betrug 2,25 % Estradiol und 8,45 % Norethisteronacetat. Die Hilfsstoffe wurden in Mengen von 1 und 5 % zugesetzt. Die Lagerung erfolgte bei Raumtemperatur in geschlossenen Polyethylenbeuteln.

Tabelle 1

| Einfluß verschiedener Hilfsstoffe auf die Rekristallisationszeit von Estradiol und Norethisteronacetat bei Raumtemperatur. | | |
|---|---|---|
| **Hilfsstoff** | **Konzentration** | **Dauer d. Kristallisation** |
| --- | --- | < 3 Wochen |
| Polyethylen (20)- | 1% | < 3 Wochen |
| sorbitanmonooleat | 5% | < 1 Woche |
| Na-laurylsulfat | 1% | < 3 Wochen |
| | 5% | < 1 Woche |
| Saccarosemono- | 1% | < 3 Wochen |
| myristat | 5% | < 2 Wochen |
| Ölsäureoleylester | 1% | < 4 Wochen |
| | 5% | < 8 Wochen |
| Olivenöl | 1% | < 3 Wochen |
| | 5% | < 5 Wochen |
| Sojaöl | 1% | < 3 Wochen |
| | 5% | < 5 Wochen |
| Propylenglycol | 1% | < 3 Wochen |
| | 5% | < 2 Wochen |
| Glycerin | 1 % | < 3 Wochen |
| | 5 % | < 1 Woche |
| PEG 400 | 1 % | < 3 Wochen |
| | 5% | < 1 Woche |
| Diethylenglycol | 1 % | < 12 Wochen |
| (Transcutol) | 5% | < 4 Wochen |

**Vergleichsbeispiel 1**

Die in vitro Hautpermeation über 72 h, gemessen an der isolierten Haut von weiblichen Nacktmäusen, liegt bei dem stabilsten System mit 1 % Transcutol, das als Resorptionsförderer beschrieben ist, deutlich über der eines handelsüblichen Systems mit dem Resorptionsförderer Ethanol (Tab. 2). Das Kristallisationsverhalten ist jedoch unbefriedigend, so daß kein lagerstabiles Pflaster erhalten werden kann. Die Klebeeigenschaften dieses Pflasters sind gut, in Einzelfällen kommt es jedoch zu leichten Hautrötungen nach dem Entfernen der Pflaster.

**Beispiel 1**

Bei der Suche nach Zusatzstoffen zur Vermeidung der Hautrötungen wurde zunächst natürliches $\alpha$-Tocopherol (z. B. Copherol, Henkel), das für seine Hautschutzfunktion aus der Kosmetik bekannt ist, in die Klebematrix eingearbeitet. Dabei wurde völlig unerwartet gefunden, daß sich unter dem Zusatz unterschiedlicher Mengen $\alpha$-Tocopherol die Wirkstoffkonzentrationen in der Klebematrix deutlich erhöhen ließen, ohne daß im Beobachtungszeitraum Rekristallisationen auftraten (**Tab. 3**).

Tabelle 3

| Einfluß von $\alpha$-Tocopherol auf die Rekristallationszeit von Estradiol und Norethisteronacetat. Matrixkleber wie **Tab. 1**. | | |
|---|---|---|
| Konzentrationen Wirkstoffe | Konzentration $\alpha$-Tocopherol | Dauer d. Kristallation |
| 2,25 % / 8,45 % Estradiol / NETA | 1 % | < 4 Wochen |
| 2,25 % / 8,45 % Estradiol / NETA | 5 % | > 20 Wochen |
| 2,5 % / 10 % Estradiol / NETA | 10 % | > 20 Wochen |
| NETA: = Norethisteronacetat | | |

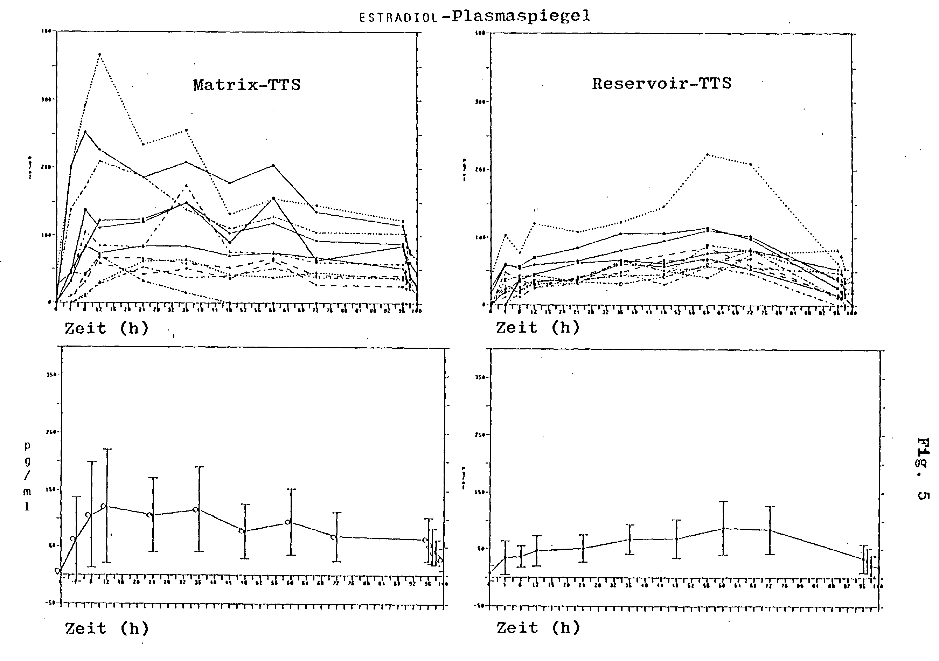

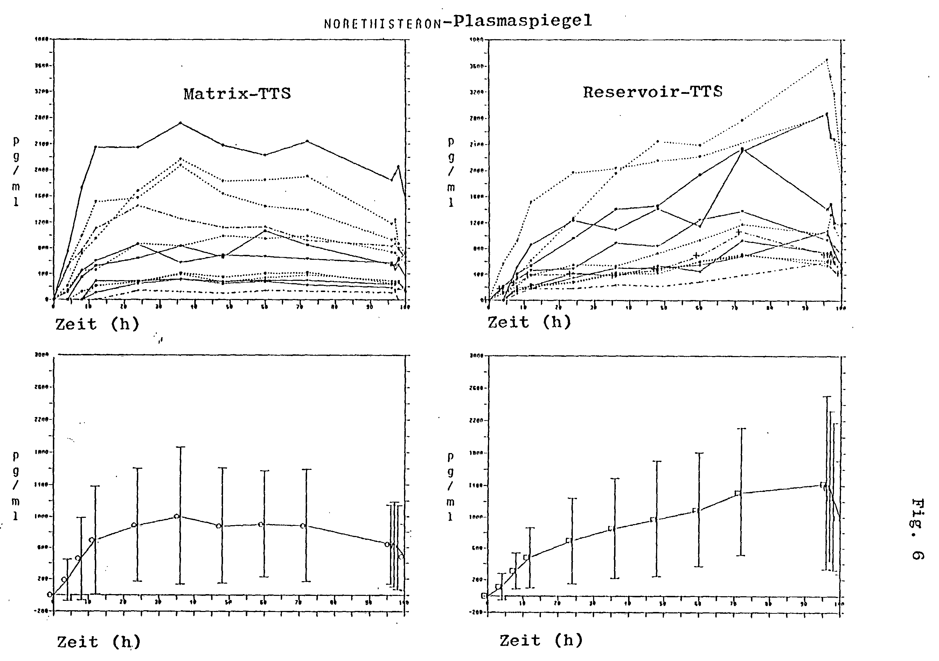

Die in vitro-Hauptpermeationsdaten der 2,25 / 8,45 % und 2,5 10 % Zubereitungen jeweils mit 5 und 10 % $\alpha$-Tocopherol sind in **Tab. 4** zusammengefaßt. Fig. 4 zeigt Estradiol-Plasmaspiegel und Fig. 5 NETA-Plasmaspiegel erfindungsgemäßer Matrixpflaster jeweils im Vergleich mit einem handelsüblichen ethanolhaltigen Reservoirsystem.

Tabelle 4

| In vitro Hautpermeation eines Matrixpflasters mit 5 % und 10 % $\alpha$-Tocopherol im Vergleich zu einem handelsüblichen ethanolhaltigen Pflaster vom Reservoirtyp, Matrixkleber wie **Tab. 1**. | | |
|---|---|---|
| Pflaster | Wirkstoffmenge / 48 h / cm$^2$ | |
| | Estradiol | Norethisteronacetat |
| Matrix 5 % | 9,3 $\mu$g | 15,8 $\mu$g |
| Matrix 10 % | 10,3 $\mu$g | 21,4 $\mu$g |
| Reservoir | 2,7 $\mu$g | 10,9 $\mu$g |

Damit liegen die in 48 Stunden durch 1 cm$^2$ hautpermeierten Mengen der Wirkstoffe bei dem 5 % $\alpha$-tocopherolhaltigen Pflaster in der gleichen Größe wie bei dem transcutolhaltigen Pflaster. Durch Steigerung der $\alpha$-Tocopherolmenge kann die Konzentration der Wirkstoffe im Pflaster gesteigert werden, und damit nimmt deutlich die in-vitro-Hautpermeation zu. Dieser Vergleich zeigt, daß Pflaster, die $\alpha$-Tocopherol enthalten, ebenfalls hohe Permeationseigenschaften besitzen. Die Permeationen liegen für beide Wirkstoffe höher als von einem handelsüblichen ethanolhaltigen Reservoirsystem. Darüberhinaus ist D-$\alpha$-Tocopherol ein sehr effizienter, die Rekristallation unterdrückender Stoff, der nahezu eine Verdoppelung der Konzentration der Wirkstoffe in der Klebematrix erlaubt.

**Beispiel 2**

Zusammensetzung

| Chlonidin | 7,4 g |
|---|---|
| $\alpha$-Tocopherol (Copherol F 1300) | 10,0 g |
| Durotak 1753 (126 - 1753) | 41,3 g |
| Durotak 2287 | 41,3 g |

Man löste die Rohstoffe, trug sie auf eine silikonisierte Folie zu einem Matrix-Flächengewicht von 96 g/m$^2$ auf, wobei man ein Laminat entsprechend **Beispiel 1** herstellte und 10 cm$^2$ große Pflaster (TTS) ausstanzte.

Danach wurde die in-vitro-Dissolution bestimmt. Prüfbedingungen: Paddle over Disk, demineralisiertes Wasser, 32 °C, 900 ml, 50 U/min, Mittelwert aus vier Freisetzungen.

| Zeit [h] | 0 | 1 | 2 | 4 | 8 | 24 | 32 | 48 |
|---|---|---|---|---|---|---|---|---|
| Freisetzung [ mg/10 cm$^2$ TTS] | 0 | 0,43 | 0,62 | 0,91 | 1,35 | 2,48 | 2,85 | 3,67 |

**Beispiel 3**

Zusammensetzung

| Selegilin | 20 g |
|---|---|
| $\alpha$-Tocopherol (Copherol F 1300) | 20 g |
| Durotak 1753 (126 - 1753) | 60 g |

Für die Herstellung von Pflastern mit einem Maxtrix-Flächengewicht von 90 g/m$^2$ folgte man **Beispiel 1.**

Danach wurde die in-vitro-Dissolution bestimmt. Prüfbedingungen: Paddle over Disk, demineralisiertes Wasser, 32 °C, 900 ml, 50 U/min, Mittelwert aus vier Freisetzungen.

| Zeit [h] | 0 | 2 | 4 | 6 |
|---|---|---|---|---|
| Freisetzung [mg/20 cm$^2$ TTS] | 0 | 14,3 | 18,8 | 20,5 |

Außerdem wurde die in-vitro-Hautpermeation bestimmt. Prüfbedingungen: Modifizierte Franz-Zelle, Mäusehaut, Akzeptor 0,9 % NaCl und 0,5 % NaN$_3$ in Wasser, Mittelwert aus zwei Zellen.

| Zeit [h] | 0 | 4 | 8 | 12 | 16 | 20 | 45 |
|---|---|---|---|---|---|---|---|
| permeierte Menge [µg/2,5 cm$^2$ TTS] | 7 | 140 | 252 | 649 | 775 | 890 | 1570 |

**Vergleichsbeispiel 1**

Die in-vitro-Hautpermeation über 72 h, gemessen an der isolierten Haut von weiblichen Nacktmäusen, liegt bei dem stabilsten System mit 1 % Transcutol, das als Resorptionsförderer beschrieben ist, deutlich über der eines handelsüblichen Systems mit dem Resorptionsförderer Ethanol **(Tab. 2).** Das Kristallationsverhalten ist jedoch unbefriedigend, so daß kein lagerstabiles Pflaster erhalten werden kann. Die Klebeeigenschaften dieses Pflasters sind gut, in Einzelfällen kommt es jedoch zu leichten Hautrötungen nach dem Entfernen der Pflaster.

Tabelle 2

| In vitro Hautpermeation eines Pflasters mit 1 % Transcutol im Vergleich zu einem handelsüblichen ethanolhaltigen Pflaster vom Reservoirtyp. Matrixkleber wie **Tab. 1**. | | |
|---|---|---|
| Pflastertyp | Wirkstoffmenge / 72 h / cm$^2$ | |
| | Estradiol | Norethisteronacetat |
| Matrix | 13,5 µg | 17,8 µg |
| Reservoir | 3,4 µg | 6,5 µg |

**Anwendungsbeispiel 1**

| Estradiol | 2,0 g |
|---|---|
| $\alpha$-Tocopherol | 5,0 g |
| Durotak 1753 | 75,0 g |

(fortgesetzt)

| | |
|---|---|
| Bio-PSA X7-4602 | 18,0 g |

Die vorstehend angegebenen Rohstoffe werden bis zur klaren Lösung gemischt. Die Lösung wird auf eine silikonisierte Folie oder Papier gestrichen, so daß ein Flächengehalt von 100 g/m$^2$ resultiert. Auf die getrocknete Matrix wird eine transparente Polypropylen- oder Polyesterfolie laminiert. Aus dem Laminat werden die fertigen Pflaster mit Größen zwischen 10 cm$^2$ (entsprechend 2 mg Wirkstoff) oder 40 cm$^2$ (entsprechend 8 mg Wirkstoff) gestanzt.

Die Beispiele zeigen, daß durch die günstigen Wirkungen von α-Tocopherol in Klebestoffmatrices mehrere erwünschte Effekte erreicht werden:

- α-Tocopherol erhöht die Klebkraft schlecht klebender Matrices
- α-Tocopherol ist als Hautschutzfaktor wirksam
- α-Tocopherol erhöht die Löslichkeit von Wirkstoffen in Matrices über das Maß von herkömmlichen ölen hinaus
- α-Tocopherol erniedrigt den Wassergehalt der Klebstoffmatrices
- α-Tocopherol erhöht die thermodynamische Aktivität von Wirkstoffen in hydratisierten Matrices
- α-Tocopherol kann die Hautpermeation schlecht permeierender Wirkstoffe erhöhen und damit auch potentiell hautschädigende Resorptionsförderer vernichten.

**Anwendungsbeispiel 2**

| | |
|---|---|
| Estradiol | 2,5 g |
| α-Tocopherol | 10,0 g |
| Durotak 1753 | 88,0 g |
| Bio-PSA X7-4602 | 7,5 g |

Die Rohstoffe werden wie in Anwendungsbeispiel 1 zu einem Laminat verarbeitet. Die Pflastergrößen betragen 8 cm$^2$ (für 2 mg Estradiol) und 32 cm$^2$ (für 8 mg Estradiol). Das Matrix-Flächengewicht beträgt hierbei 100 g/m$^2$.

**Anwendungsbeispiel 3 und Vergleichsanwendungsbeispiel 1**

| | |
|---|---|
| Estradiol | 2,5 g |
| α-Tocopherol | 7,5 g |
| Durotak 1753 | 90,0 g |

Die Rohstoffe werden wie in Beispiel 1 zu einem Laminat verarbeitet. Die Pflastergrößen betragen 10 cm$^2$ (für 2 mg Estradiol), 20 cm$^2$ (für 4 mg Estradiol) und 40 cm$^2$ (für 8 mg Estradiol) bei einem Matrix-Flächengewicht von 80 g/cm$^2$. Die in-vitro-Hautpermeation ist in **Tab. 5** im Vergleich zu einem handelsüblichen Estradiol-Reservoir Pflaster dargestellt. Konzentration des Matrix-Pflasters: 2,5 % Estradiol.

| Pflaster | Wirkstoffmenge / 48 h / cm$^2$ |
|---|---|
| | Estradiol |
| Matrix 2,5 % | 9,7 µg |
| Reservoir | 5,2 µg |

Außerdem wurden die Blutspiegelverläufe bei einem erfindungsgemäßen Matrix-Pflaster (20 cm$^2$) und einem handelsüblichen Estradiol-Reservoir-Pflaster ermittelt. Diese Studie wurde an sechs gesunden Probandinnen durchgeführt. Im randomisierten cross-over-Versuch wurden je ein Matrix- bzw. Reservoir-Pflaster über vier Tage appliziert, wobei die Blutspiegelverläufe durch RIA (Radioimmunassay) ermittelt wurden. Die Basis-Estradiol-Blutspiegel vor Applikation der Pflaster wurden von den aktuellen Werten subtrahiert.

Die statistische Auswertung der Daten (**Fig. 1**), insbesondere der Flächen unter den Kurven, die die insgesamt absorbierte Estradiolmenge angeben, ergab keine signifikanten Unterschiede zwischen beiden Formulierungen. Das erfindungsgemäße Matrix-Pflaster zeichnete sich jedoch durch wesentlich gleichmäßigere und reproduzierbare Blutspiegel aus, die im Vergleich zum Reservoir-Pflaster über den gesamten Applikationszeitraum konstant anhielten. Durch einen Vergleich der Symbole in **Fig. 1** läßt sich erkennen, daß Probandinnen mit geringen Blutspiegeln diese bei beiden Applikationsarten aufwiesen.

**Anwendungsbeispiel 4 und Vergleichsanwendungsbeispiel 2**

Zusammensetzung

| | |
|---|---|
| Estradiol 2,5 % | 2,5 g |
| Norethisteronacetat (NETA; 10 %) | 10,0 g |
| $\alpha$-Tocopherol | 10,0 g |
| Durotak 1753 | auf 100 g |

Es wurden erfindungsgemäße Pflaster wie beim Anwendungsbeispiel 3 mit der Ausnahme hergestellt, daß die Pflastergröße 33 cm$^2$ betrug.

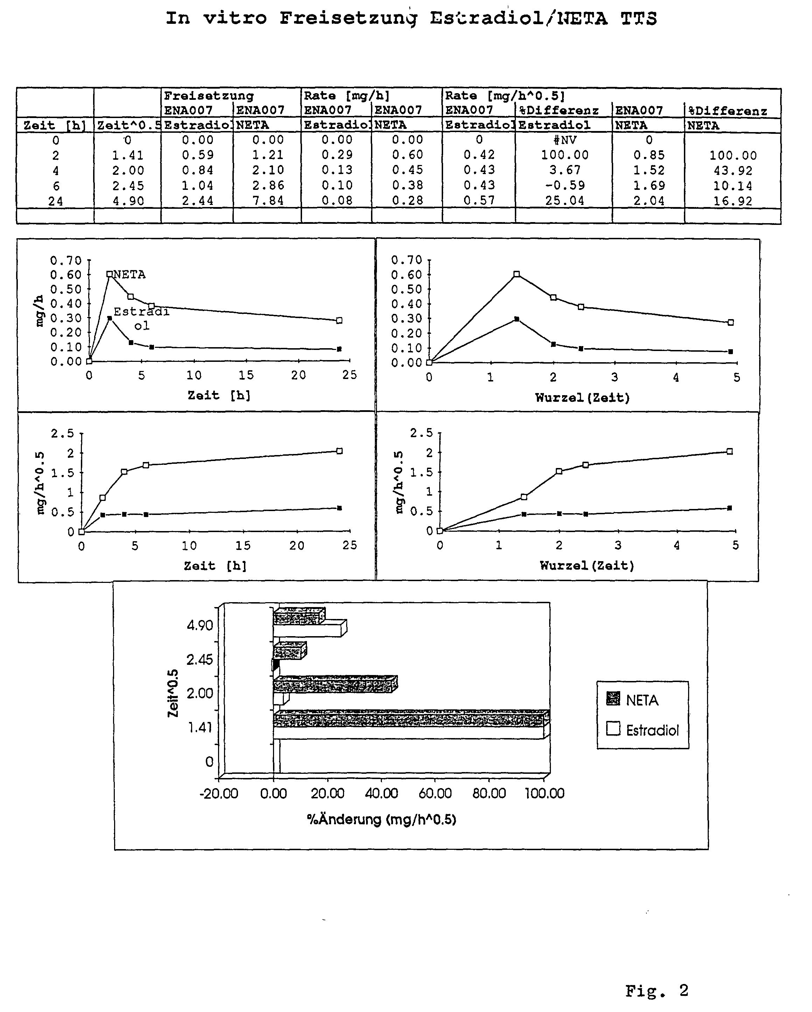

Die in-vitro-Freisetzung wurde gemäß Beispiel 3 durchgeführt, wobei die ermittelten Ergebnisse in **Fig. 2** graphisch dargestellt sind. Im allgemeinen ist die Freisetzung von Wirkstoffen [mg/h$^{1/2}$] im Verlauf der Zeit [h] konstant. Im vorliegenden Fall ergab sich jedoch überraschenderweise, daß die Freisetzung im Verlauf der Zeit nicht konstant ist, vielmehr eine überproportionale Freisetzung zu beobachten ist.

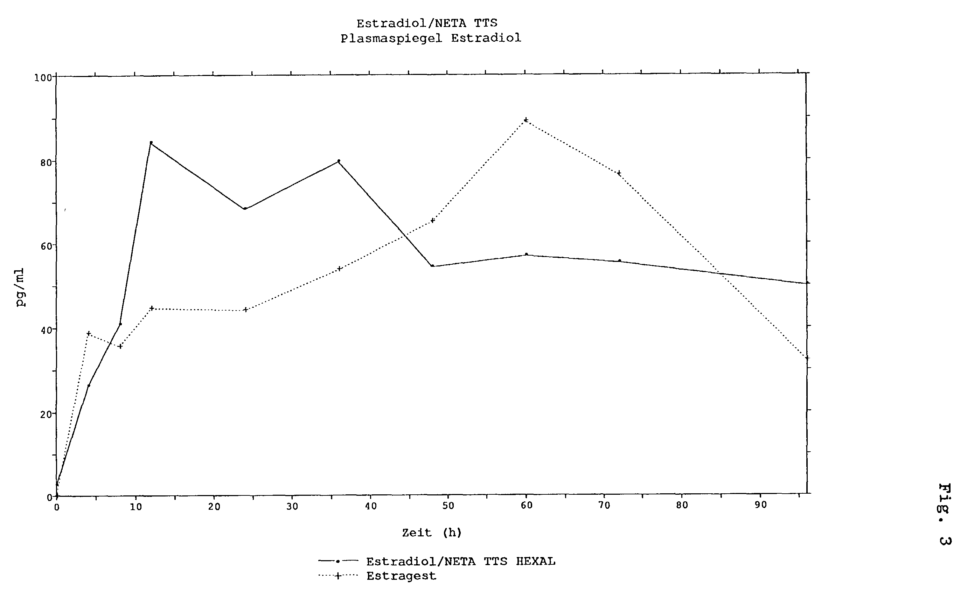

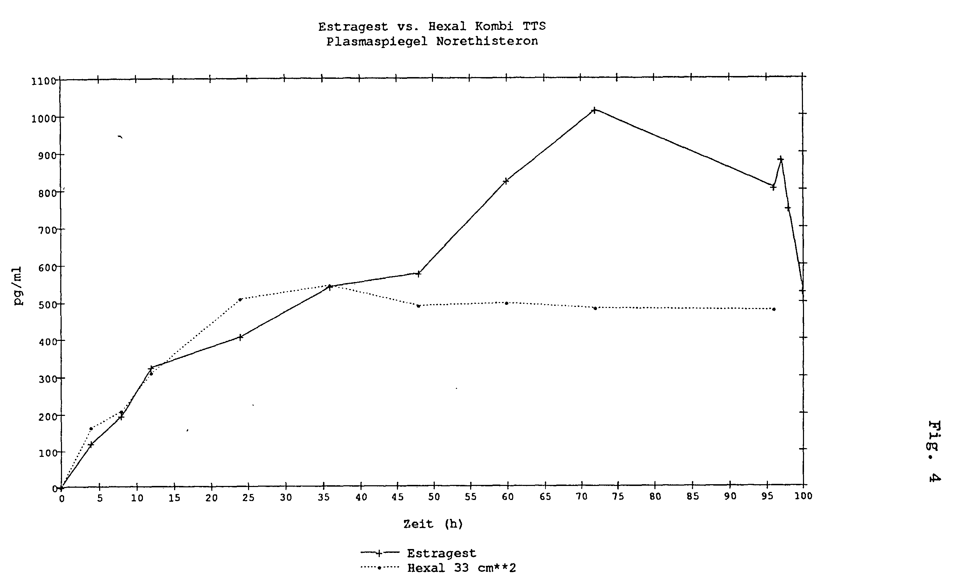

Außerdem wurden Blutspiegelverläufe für ein erfindungsgemäßes Kombinations-Pflaster und ein Kombinations-Pflaster des Handels ermittelt, wobei zur Durchführung auf Anwendungsbeispiel 3 verwiesen werden kann. **Fig. 3** ist der Estradiol-Plasmaspiegel (Punkte für erfindungsgemäßes Pflaster und Kreuze für Vergleichspflaster) und **Fig. 4** der NETA-Plasmaspiegel (Punkte für erfindungsgemäßes Pflaster und Kreuze für Vergleichspflaster) zu entnehmen. Den Figuren 3 und 4 kann man entnehmen, daß sich beim erfindungsgemäßen Pflaster nach etwa 45 bis 50 h ein konstanter Plasmaspiegel einstellt, der mit dem Vergleichspflaster nicht erreicht wird. Zusätzlich wurden beim Vergleichspflaster eine Hautzerstörung und eine massive Hautirritation beobachtet.

**Vergleichsanwendungsbeispiel 3**

| | |
|---|---|
| Estradiol | 2,25 g |
| $\alpha$-Tocopherol | 5,00 g |
| Norethisteronacetat | 8,45 g |
| Durotak 1753 | 66,97 g |
| Bio-PSA X7-4602 | 22,33 g |

Die Rohstoffe werden wie in Beispiel 1 gelöst und auf eine silikonisierte Folie zu einem Flächengewicht von 88,9 g/m$^2$ der getrockneten Matrix verarbeitet. Aus dem Laminat werden Pflaster von 10 cm$^2$ (entsprechend 2 mg Estradiol/7,5 mg NETA), 20 cm$^2$ (entsprechend 4 mg Estradiol/15 mg NETA) oder 50 cm$^2$ (entsprechend 10 mg Estradiol/37,5 mg NETA) gestanzt.

**Vergleichsanwendungsbeispiel 4**

| | |
|---|---|
| Norethisteronacetat | 10,0 g |
| Durotak 1753 | 87,0 g |
| $\alpha$-Tocopherol | 10,0 g |

Die Rohstoffe werden gelöst und auf eine silikonisierte Folie zu einem Matrix-Flächengewicht von 80 g/m$^2$ aufgetragen. Wie in Beispiel 1 wird hieraus ein Laminat hergestellt und zu 20 cm$^2$ (entsprechend 16 mg NETA) großen Pflastern gestanzt.

**Patentansprüche**

**1.** Wirkstoffpflaster in Form eines Laminats, umfassend einen Träger und eine Matrix aus einem einzigen Polymeren und mit einem Gehalt an Vitamin E oder einem Vitamin E-Derivat sowie mindestens einem Wirkstoff zur Behandlung von Parkinsonismus, wobei

- es sich bei dem Polymeren um ein Copolymerisat auf Acrylatbasis handelt und
- das Polymere auf Acrylatbasis aus mindestens 50 Gew.-% Alkylacrylat-Monomer, umfassend 2-Ethylhexylacrylat, und gegebenenfalls Acrylat-Monomer, copolymerisiert mit Acrylsäure, besteht.

**2.** Wirkstoffpflaster nach Anspruch 1, **gekennzeichnet durch** Selegelinsalze, Selegelinbase, Bromocriptin oder Lisurid als Wirkstoff.

**3.** Wirkstoffpflaster nach Anspruch 1 oder 2, **gekennzeichnet durch** eine derartige Konzentration des Wirkstoffs oder zumindest eines der Wirkstoffe, daß bei Applikation des Pflasters auf die Haut die Sättigungskonzentration des Wirkstoffs oder mindestens eines der Wirkstoffe überschritten wird.

**4.** Wirkstoffpflaster nach Anspruch 3, **gekennzeichnet durch** eine Konzentration des Wirkstoffs oder zumindest eines der Wirkstoffe, die im Lagerzustand des Wirkstoffpflasters der Sättigungskonzentration angenähert ist.

**5.** Wirkstoffpflaster nach Anspruch 3 oder 4, **dadurch gekennzeichnet, daß** die Sättigungskonzentration bei Applikation des Pflasters im Bereich von 5 bis 100 und insbesondere 20 bis 80 % überschritten wird.

**6.** Wirkstoffpflaster nach einem der vorhergehenden Ansprüche, **gekennzeichnet durch** einen Träger in Form einer Folie mit begrenzter oder ohne Wasser- und Wasserdampfdurchlässigkeit.

**7.** Wirkstoffpflaster nach einem der vorhergehenden Ansprüche, **gekennzeichnet durch** alpha-Tocopherol, alpha-Tocopherol-Acetat, alpha-Tocopherol-Succinat und/oder alpha-Tocopherol-Nikotinat als Vitamin E-Deriviat.

**8.** Wirkstoffpflaster nach einem der vorhergehenden Ansprüche, **gekennzeichnet durch** einen Gehalt an Vitamin E und/oder Vitamin E-Derivat im Bereich von 0,5 bis 20 und insbesondere 1 bis 100 Gew.-% auf Basis der Matrix.

**9.** Wirkstoffpflaster nach einem der vorhergehenden Ansprüche, **gekennzeichnet durch** eine akzeptable Pflastergröße bis zu etwa 200 cm$^2$, wobei man über die Einstellung des Gehalts an Vitamin E und/oder Vitamin E-Derivat den Wirkstoffblutspiegel einem therapeutisch erwünschten Blutspiegel annähert.

**Claims**

**1.** Active ingredient patch in the form of a laminate, comprising a carrier and a matrix of a single polymer and having a content of vitamin E or a vitamin E derivative and at least one active ingredient for the treatment of parkinsonism, where

- the polymer is a copolymer based on acrylate, and
- the polymer based on acrylate consists of at least 50 % by weight of alkyl acrylate monomer including 2-ethylhexyl acrylate and optionally acrylate monomer copolymerised with acrylic acid.

**2.** Active ingredient patch according to claim 1, **characterized by** selegelin salts, selegelin base, bromocriptin or lisurid as active ingredient.

**3.** Active ingredient patch according to claim 1 or 2, **characterized by** a concentration of the active ingredient or at least one of the active ingredients such that the saturation concentration of the active ingredient or at least of one of the active ingredients is exceeded on application of the patch to the skin.

**4.** Active ingredient patch according to claim 3, **characterized by** a concentration of the active ingredient or at least of one of the active ingredients which is approximately the saturation concentration in the storage state of the active ingredient patch.

5. Active ingredient patch according to claim 3 or 4, **characterized in that** the saturation concentration on application of the patch is exceeded in the range from 5 to 100 %, and in particular 20 to 80 %.

6. Active ingredient patch according to one of the preceding claims, **characterized by** a carrier in the form of a film of limited or no permeability to water and water vapour.

7. Active ingredient patch according to one of the preceding claims, **characterized by** alpha-tocopherol, alpha-tocopherol acetate, alpha-tocopherol succinate and/or alpha-tocopherol nicotinate as the vitamin E derivative.

8. Active ingredient patch according to one of the preceding claims, **characterized by** a content of vitamin E and/or vitamin E derivative in the range from 0.5 to 20 % and in particular 1 to 100 % by weight, based on the matrix.

9. Active ingredient patch according to one of the preceding claims, **characterized by** an acceptable patch size of up to about 200 cm$^2$, the active ingredient blood level approaching a therapeutically desirable blood level via adjustment of the content of vitamin E and/or vitamin E derivative.

**Revendications**

1. Pansement à agent actif sous forme de stratifié, comprenant un support et une matrice d'un seul polymère et ayant une teneur en vitamine E ou en dérivé de vitamine E ainsi qu'au moins un agent actif pour traiter le parkinsonisme, dans lequel

- quant au polymère, il s'agit d'un copolymère à base d'acrylate et
- le polymère à base d'acrylate est constitué au moins de 50 % en poids de monomère acrylate d'alkyle, comprenant l'acrylate de 2-éthylhexyle, et éventuellement un monomère acrylate, copolymérisé avec l'acide acrylique.

2. Pansement à agent actif selon la revendication 1, **caractérisé par** les sels de sélégéline, une base de sélégéline, la bromocryptine ou le lisuride en tant qu'agent actif.

3. Pansement à agent actif selon la revendication 1 ou 2, **caractérisé par** une telle concentration de l'agent actif ou d'un des agents actifs et en ce que la concentration de saturation de l'agent actif ou d'au moins un des agents actifs est dépassée lors de l'application du pansement sur la peau.

4. Pansement à agent actif selon la revendication 3, **caractérisé par** une concentration de l'agent actif ou d'un des agents actifs qui s'approche de la concentration de saturation à l'état entreposé du pansement à agent actif.

5. Pansement à agent actif selon la revendication 3 ou 4, **caractérisé en ce que** lors de l'application du pansement à raison de 5 à 100 % et en particulier de 20 à 80 %, la concentration de saturation est dépassée.

6. Pansement à agent actif selon l'une quelconque des revendications précédentes, **caractérisé par** un support sous forme d'une feuille n'ayant aucune perméabilité voire une perméabilité limitée à l'eau et à la vapeur d'eau.

7. Pansement à agent actif selon l'une quelconque des revendications précédentes, **caractérisé par** l'alpha-tocophérol, l'acétate d'alpha-tocophérol, le succinate d'alpha-tocophérol et/ou le nicotinate d'alpha-tocophérol en tant que dérivé de vitamine E.

8. Pansement à agent actif selon l'une quelconque des revendications précédentes, **caractérisé par** une teneur en vitamine E et/ou en dérivé de vitamine E dans la plage de 0,5 à 20 % et en particulier de 1 à 100 % en poids par rapport à la matrice.

9. Pansement à agent actif selon l'une quelconque des revendications précédentes, **caractérisé par** une taille acceptable de pansement allant jusqu'à environ 200 cm$^2$, où l'on rapproche la concentration sanguine de l'agent actif d'une concentration sanguine thérapeutique souhaitée en ajustant la teneur en vitamine E et/ou en dérivé de vitamine E.

Fig. 1

# In vitro Freisetzung Estradiol/NETA TTS

| Zeit [h] | Zeit^0.5 | Freisetzung | | Rate [mg/h] | | Rate [mg/h^0.5] | | | |
|---|---|---|---|---|---|---|---|---|---|
| | | ENA007 Estradiol | ENA007 NETA | ENA007 Estradiol | ENA007 NETA | ENA007 Estradiol | %Differenz Estradiol | ENA007 NETA | %Differenz NETA |
| 0 | 0 | 0.00 | 0.00 | 0.00 | 0.00 | 0 | #NV | 0 | |
| 2 | 1.41 | 0.59 | 1.21 | 0.29 | 0.60 | 0.42 | 100.00 | 0.85 | 100.00 |
| 4 | 2.00 | 0.84 | 2.10 | 0.13 | 0.45 | 0.43 | 3.67 | 1.52 | 43.92 |
| 6 | 2.45 | 1.04 | 2.86 | 0.10 | 0.38 | 0.43 | -0.59 | 1.69 | 10.14 |
| 24 | 4.90 | 2.44 | 7.84 | 0.08 | 0.28 | 0.57 | 25.04 | 2.04 | 16.92 |

Fig. 2

Estradiol/NETA TTS
Plasmaspiegel Estradiol

Zeit (h)

——•—— Estradiol/NETA TTS HEXAL
·····+····· Estragest

EP 0 813 865 B1

Fig. 3

Estragest vs. Hexal Kombi TTS
Plasmaspiegel Norethisteron

Zeit (h)

—+— Estragest
·····•····· Hexal 33 cm**2

EP 0 813 865 B1

18

Fig. 4

Fig. 5

ESTRADIOL-Plasmaspiegel

Reservoir-TTS

Matrix-TTS

Zeit (h)

Zeit (h)

Zeit (h)

Zeit (h)

Fig. 6